Europäisches Patentamt

European Patent Office

Office européen des brevets

(19)

(11) Veröffentlichungsnummer: **0 137 492**
**A2**

(12) **EUROPÄISCHE PATENTANMELDUNG**

(21) Anmeldenummer: **84112153.6**

(22) Anmeldetag: **10.10.84**

(51) Int. Cl.⁴: **C 12 Q 1/48**
**G 01 N 33/577, C 12 P 1/00**
**C 12 N 15/00, C 07 K 15/00**

(30) Priorität: **10.10.83 DE 3336786**

(43) Veröffentlichungstag der Anmeldung:
**17.04.85 Patentblatt 85/16**

(84) Benannte Vertragsstaaten:
**AT BE CH DE FR GB IT LI NL SE**

(71) Anmelder: **GÖDECKE AKTIENGESELLSCHAFT**
**Salzufer 16**
**D-1000 Berlin 10(DE)**

(72) Erfinder: **Lenger, Karin, Dr.**
**Brucknerstrasse 4**
**D-2400 Lübeck(DE)**

(54) Verfahren zur Frühdiagnose maligner Tumoren bei Säugetieren und Menschen.

(57) Es wird ein Verfahren zur Frühdiagnose maligner Tumoren bei Säugetieren und Menschen beschrieben, bei dem man die im Blut vorhandenen Enzymformen der Nukleosid-Nukleotid-Phosphotransferasen bestimmt und das erhaltene Enyzmmuster mit einem Standardmuster, erhalten aus dem Blut entsprechender gesunder Individuen, vergleicht. Zum direkten Nachweis der für maligne Tumoren spezifischen Enzymform(en) im Blut erkrankter Individuen wird ein immunologisches Nachweisverfahren vorgeschlagen, das von gegen die spezifische(n) Enzymform(en) gerichteten monoklonalen oder polyklonalen Antikörpern Gebrauch macht.

EP 0 137 492 A2

Croydon Printing Company Ltd.

## Beschreibung

Die Erfindung betrifft ein Verfahren zur Frühdiagnose maligner Tumoren bei Säugetieren und Menschen. Der Begriff "Frühdiagnose" bezeichnet erfindungsgemäß den Nachweis maligner Tumoren zu einem Zeitpunkt, zu dem diese noch durch keinerlei wahrnehmbare Symptome in Erscheinung getreten sind.

Es ist bereits bekannt, bestimmte maligne Tumoren durch Analyse des Blutserums anhand von im Blut befindlichen Substanzen, sogenannten "Tumormarkern" nachzuweisen, die Antigeneigenschaften aufweisen. Diese Substanzen können im Serum mit Hilfe von monoklonalen Antikörpern nachgewiesen werden (vergl. Biologie in unserer Zeit 14, 97 bis 102 (1984). Bisher sind tumorspezifische Substanzen jedoch nur für wenige Tumorarten, wie beispielsweise gastrointestinale Tumoren, bekannt und deren Nachweis ist erst in einem Stadium möglich, in dem der Tumor ausreichende Mengen des Stoffwechselproduktes ausscheidet, d.h. wenn das Tumorwachstum bereits fortgeschritten ist. Darüberhinaus hat sich dieses Verfahren als unzuverlässig erwiesen, da es sowohl zu falschen positiven als auch zu falschen negativen Ergebnissen führt. Bisher war es nicht möglich, die Entstehung maligner Tumoren bereits zu Beginn des Tumorwachstums und unabhängig von der Lokalisation im Körper durch Analyse des Blutplasmas oder -serums zu erfassen.

Es konnte im Rahmen wissenschaftlicher Forschungsarbeiten zur Untersuchung des Tumorwachstums bei Ratten mit malignen Lebertumoren (Morris hepatoma 9121) gezeigt werden, daß in den Zellkernen erkrankter Zellen reproduzier-

bar Enzymformen der Nukleosid-Nukleotid-Phosphotransferasen auftreten, welche in den Kernen entsprechender gesunder Zellen fehlen (K. Lenger, Int. J. Biochem. 14, 53 bis 61 und 673 bis 678 (1982) sowie 15, 383 - 393 (1983). Es wird vermutet, daß die in den Kernen turmorkranker Zellen zusätzlich auftretenden Enzymformen der Nukleosid-Nukleotid-Phosphotransferasen in verstärktem Umfang die bei der Zellproliferation benötigten Bausteine zur Verfügung stellen.

Überraschend konnte nun festgestellt werden, daß derartige Nukleosid-Nukleotid-Phosphotransferasen auch im Blut von Säugetieren und Menschen nachweisbar sind und daß das Enzymmuster entsprechender gesunder und tumorkranker Individuen sich in analoger Weise voneinander unterscheidet, wie dies an den Zellkernen von Rattenlebern nachgewiesen werden konnte. Im Blut erkrankter Individuen treten reproduzierbar Enzymformen der Nukleosid-Nukleotid-Phosphotransferasen auf, welche im Blut gesunder Individuen fehlen. Die erfindungsgemäß nachgewiesenen Enzyme sind als NTP:NDP:NMP - Nukleosid-Nukleotid-Phosphotransferasen definiert (K. Lenger, Int. J. Biochem. 14, 673 bis 677 (1982)). Durch Bestimmung der verschiedenen Enzymformen der Nukleosid-Nukleotid-Phosphotransferasen ist es möglich, das Vorhandensein maligner Tumoren im Frühstadium durch Analyse des Blutplasmas oder -serums nachzuweisen.

Es wird demgemäß ein Verfahren zur Frühdiagnose maligner Tumoren bei Säugetieren und Menschen vorgeschlagen, welches dadurch gekennzeichnet ist, daß man die im Blut vorhandenen Enzymformen der Nukleosid-Nukleotid-Phosphotransferasen bestimmt und das erhaltene Enzymmuster mit einem Standardmuster, erhalten aus dem Blut entsprechender gesunder Individuen, vergleicht.

Zur Vereinfachung des Verfahrens beispielsweise für Serienuntersuchungen wird erfindungsgemäß ferner ein immunologisches Nachweisverfahren vorgeschlagen, das von monoklonalen oder polyklonalen Antikörpern Gebrauch macht, die gegen die für maligne Tumoren spezifische(n) Enzymform(en) gerichtet sind.

Das erfindungsgemäße Verfahren kann durchgeführt werden, indem man frisch entnommenes Blut zunächst mit Citrat versetzt und anschließend zentrifugiert, um das Plasma zu gewinnen. Aus diesem wird durch Ammoniumsulfat-Fällung und anschließende Zentrifugation der enzymhaltige Niederschlag isoliert. Dieser wird wieder gelöst und zur Fraktionierung auf eine Chromatographiesäule aufgetragen. Durch Elution mit einem kontinuierlichen NaCl-Gradienten werden die einzelnen Enzymfraktionen erhalten, die durch Bestimmung des Proteingehaltes und der phosphorylierenden Aktivität nach bekannten Verfahren ausgewertet werden.

Für die Fraktionierung kann ferner jedes bekannte und geeignete Verfahren, insbesondere die Hochdruck-Flüssigchromatographie (HPLC) verwendet werden.

In einer bevorzugten Ausführungsform der Erfindung werden die einzelnen Aufarbeitungsstufen in 5 bis 15 mM, vorzugsweise 10 mM Morpholinopropansulfonsäure (MOPS)-Puffer durchgeführt, dessen pH-Wert 6,9 bis 8, vorzugsweise 7,5 beträgt, und der ferner Dithiothreit (DTE) in Mengen von 0,2 bis 10 mM, vorzugsweise o,5 mMol enthält.

Die Fällung des Enzymproteins aus dem Blutplasma oder Serum wird vorzugsweise in der Weise durchgeführt, daß man eine gesättigte Ammoniumsulfatlösung in MOPS-Puffer mit dem Blutplasma im Verhältnis 2 : 1 vermischt und den Niederschlag nach einiger Zeit abzentrifugiert. Der in MOPS-Puffer gelöste Niederschlag wird unmittelbar anschließend auf eine Chromatographiesäule gegeben.

Als Säulenmaterial hat sich insbesondere Diethylaminoethylcellulose (DEAE-Cellulose) als geeignet erwiesen. Die Elution der verschiedenen Enzymformen wird mit einem linearen NaCl-Gradienten von 0,0 bis 1,0 M in MOPS-Puffer durchgeführt. Hierbei hat sich eine Pumpgeschwindigkeit von etwa 60 ml/h besonders bewährt. Die Fraktionsgröße beträgt jeweils etwa 0,5 bis 1 ml, wobei sich der gesamte Elutionsvorgang über einen Zeitraum von etwa 8 Stunden erstreckt und das Waschen der Säule mit MOPS-Puffer, die Elution mit dem NaCl-Gradienten und das Nachwaschen mit 1 M Natriumchlorid in MOPS-Puffer einschließt.

Zur Messung der Enzymaktivität ist ein äußerst empfindliches Testverfahren erforderlich. Als Substrat wird vorzugsweise [14]C-markiertes d-Thymidin eingesetzt und die Bestimmung nach dem von Lenger in Int. J. Biochem. 14, 53 bis 61 (1982) beschriebenen Verfahren durchgeführt. Es können jedoch auch andere Nukleoside wie beispielsweise Cytidin oder Adenosin als Substrat verwendet werden.

Wird Blut gesunder und tumorkranker Patienten nach dem erfindungsgemäßen Verfahren untersucht, so zeigt sich bei dem Vergleich der erhaltenen Enzymmuster, daß bei tumorkranken Patienten reproduzierbar zwei Enzymformen auftreten, welche bei einer NaCl-Konzentration von 0,4 bis 0,5 M bzw. 0,8 bis 0,9 M von der Chromatographiesäule eluiert werden und im die Blut gesunder Patienten fehlen.

Zur Schaffung eines vereinfachten, für die Durchführung von Serienuntersuchungen geeigneten Verfahrens zum Nachweis der für maligne Tumoren spezifischen Enzymform(en) wird(werden) zunächst die bei tumorkranken Individuen zusätzlich auftretende(n) Enzymform(en) aus den jeweiligen Fraktionen isoliert.

Die Isolierung kann nach bekannten Verfahren, beispielsweise durch Ausfällen mit geeigneten Salzen, Zentrifugieren, Waschen und gegebenenfalls Trocknen oder Gefriertrocknen durchgeführt werden.

Anschließend werden mono- oder polyklonale Antikörper gegen die bei tumorkranken Individuen zusätzlich auftretende(n) Enzymform(en) hergestellt, indem man diese zur Bildung von Antikörpern befähigten Säugetieren, vorzugsweise Mäusen oder Meerschweinchen, als Antigen appliziert und nach bekannten Verfahren mono- oder polyklonale Antikörper gewinnt (vgl. Köhler, G. und Milstein, C; Nature 256, 495 bis 497, (1975)). Diese werden anschließend in einem immunologischen Verfahren verwendet, um die als Antigen wirkende(n), im Blut tumorkranker Individuen zusätzlich vorhandene(n) Enzymform(en) der Nukleosid-Nukleotid-Phosphotransferasen nachzuweisen.

Das immunologische Nachweisverfahren gemäß der vorliegenden Erfindung kann nach verschiedenen an sich bekannten Prinzipien durchgeführt werden.

So können beispielsweise die mono- oder polyklonalen Antikörper zum Nachweis der Immunreaktion radioaktiv markiert sein, vorzugsweise mit $^{125}J$ oder $^{3}H$, insbesondere aber mit $^{125}J$. Ferner ist eine Markierung der Antikörper mit einem Enzym, wie Meerrettichperoxidase oder alkalischer

Phosphatase, vorzugsweise Meerrettichoxidase, oder mit einer fluoreszierenden Substanz wie Fluorescaim oder Fluorescein, vorzugsweise mit Fluorescein bekannt.

In einer weiteren Ausführungsform des immunologischen Nachweisverfahrens gemäß der vorliegenden Erfindung kann (können) die als Antigen wirkende(n) Enzymform(en) radioaktiv oder in sonstiger Weise wie oben angegeben markiert sein. Die Markierung der erfindungsgemäß als Antigen wirkenden Enzymform(en) kann in vivo oder in vitro durchgeführt werden. Insbesondere kann die Bereitschaft der erfindungsgemäßen Enzymform(en) zur Bildung von Komplexen mit Steroidhormonen für die Markierung genutzt werden (vgl. K. Lenger Int. J. Biochem. 15, 383 bis 393 (1983)). Für die Markierung in vivo wird das jeweilige Steroidhormon dem Patienten verabreicht, während für die Markierung in vitro das Blutplasma oder -serum mit dem Hormon inkubiert wird.

Abhängig von der gewählten Ausführungsform des immunologischen Nachweisverfahrens können die Antikörper ferner in flüssiger Phase vorliegen oder an eine feste Phase, wie die Wandung eines Untersuchungsgefässes oder leicht abtrennbare größere Partikel gebunden sein.

Nachfolgend sind einige Beispiele für bekannte erfindungsgemäß in Betracht kommende immunologische Nachweisverfahren angegeben:

1. Nachweis unter Verwendung radioaktiv markierter Antigene oder Antikörper = radio immuno assay (RIA), vgl. W.M. Hunter in "Handbook of Experimental Immunology, Kapitel 17, Oxford, Blackwell Scientific Publications, 1973. Die Durchführung kann als kompetitiver RIA oder als Immunoradiometrischer Assay (IRMA) erfolgen.

2. Nachweis mit enzymmarkierten Antigenen oder Antikörpern = enzymeimmunoassay (EIA), vgl. U. Göbel Wehrmed.
Mschr. 12, 354 (1979) Enzyme-Immunoassays. Bekannt
sind
   a) Enzyme-Linked-Immunosorbent-Assay (ELISA)
   b) Enzyme-Multiplied-Immunoassay-Technique (EMIT)

3. Nachweis mit fluorochrommarkierten Antigenen oder Antikörpern = Fluorescenceimmunoassay (FIA), vgl. B. Viel,
MTA-Journal 1, 56 (1979), Grundlagen der Immunfluoreszenz.

Die zur Durchführung des jeweiligen Nachweisverfahrens
erforderlichen Reagentien werden in einem Testkit zusammengestellt. Dieser kann neben den markierten oder unmarkierten erfindungsgemäßen Antikörpern unter anderen die
folgenden Reagentien enthalten:

1. Positive und negative Kontrollen, vorzugsweise menschliche Kontrollseren,

2. Mittel zum Abtrennen des Antigen-Antikörperkomplexes,

3. Einen zweiten radioaktiv-, enzym- oder fluorochrommarkierten Antikörper gegen den Anti-Turmorenzym-
Antikörper,

4. Substrate für Enzymreaktionen und

5. Waschlösungen, Aufbewahrungsflüssigkeiten und Verdünnungsmittel.

Wird der Nachweis mit markierten als Antigen wirkenden
Enzymen in Kombination mit unmarkierten Antikörpern vorgenommen, so kann der Testkit ferner Mittel zum in-vivo oder in-vitro Markieren der zusätzlichen Enzymformen in der Blutprobe enthalten, wie beispielsweise ein Steroidhormon zur in-vivo Applikation oder in-vitro Inkubation oder andere geeigente Mittel zum Nachweis der Immunreaktion. Abhängig von der jeweiligen Ausführungsform der Erfindung können die in dem Testkit zusammengestellten Reagentien getrocknet, gefriergetrocknet oder gelöst in Puffer oder anderen Substanzen vorliegen.

Die Erfindung wird nachfolgend anhand von Beispielen erläutert.

## Beispiel 1

Frisch entnommenes Blut in physiologischer Natriumcitratlösung wurde 10 Minuten lang bei 4000 g in einer Kühlzentrifuge zentrifugiert. Der klare hellgelbe Überstand wurde dekantiert, mit 1 N Essigsäure auf pH 4,5 eingestellt und 30 Minuten bei 17.000 g zentrifugiert. Nach Dekantieren wurde der Überstand mit konzentrierter KOH auf pH 7,5 eingestellt. Anschließend wurde 1 Volumenteil des Überstandes mit 2 Volumenteilen einer gesättigten Ammoniumsulfatlösung in 10 mM MOPS-Puffer, pH 7,5 mit einem Gehalt an 0,5 mM DTE vermischt. Die Mischung verblieb 1 Stunde lang bei etwa 5°C im Kühlraum und wurde anschließend 30 Minuten lang bei 17.000 g zentrifugiert. Der erhaltene Niederschlag wurde in MOPS-Puffer (10 mM, pH 7,5, 0,5 mM DTE) gelöst und unmittelbar anschließend auf eine DEAE-Cellulose-Säule der Abmessung 30 x 1,6 cm aufgetragen. Die Säule wurde mit einem Säulenvolumen MOPS-Puffer vorgewaschen und anschließend mit einem NaCl-Gradienten von 0,0 bis 1,0 M in MOPS-Puffer aus einem Gradientenmischer (LKB-Gradientenmischer Ultrograd 11 300) eluiert. Die Pumpgeschwindigkeit betrug 60 ml/h und die Fraktionsgröße 0,7 ml. Die Säule wurde mit einem Volumen 1 M NaCl in MOPS-Puffer nachgewaschen. Der Proteingehalt jeder Fraktion wurde nach BIO-Rad, Protein Assay, microprocedure (Instruction Manual, BIO-RAD, 1979) und die thymidinphosphorylierende Aktivität der Fraktionen nach Lenger (vgl. a.a.O.) bestimmt.

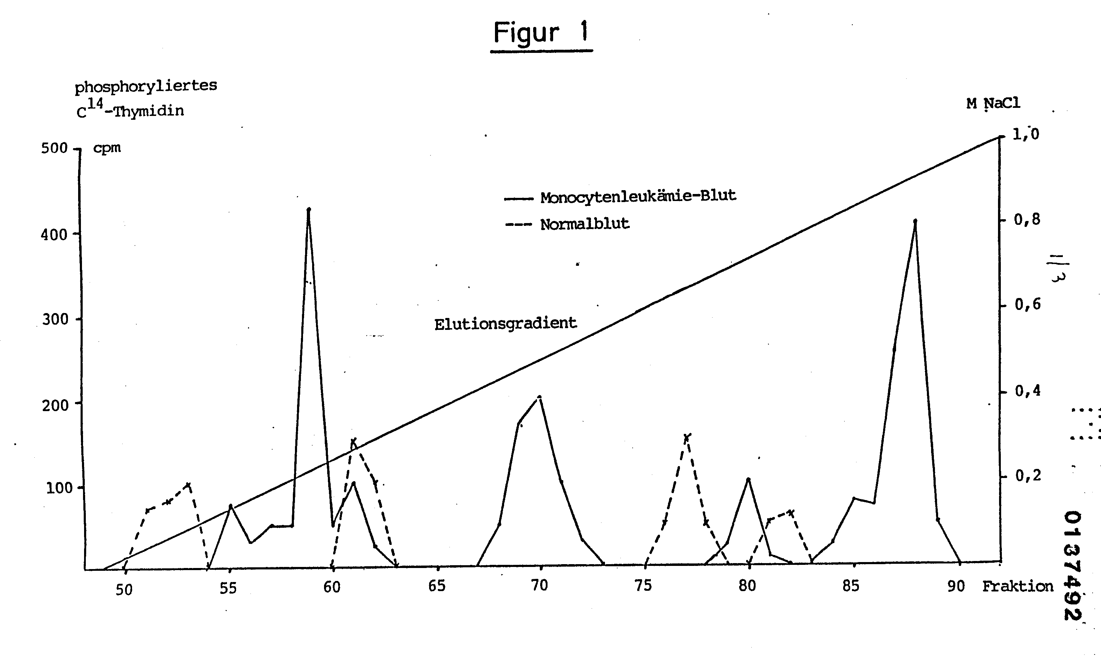

Die Ergebnisse der Bestimmungen der Nukleosid-Nukleotid-Phosphotransferasen im normalen Blutplasma sowie im Blutplasma eines an Monocytenleukämie erkrankten Patienten wurden graphisch dargestellt (vgl. Figur 1). Der Vergleich der erhaltenen Enzymmuster zeigt, daß aus dem Blut des erkrankten Patienten bei einer NaCl-Konzentration von 0,4 bis 0,5 M sowie von 0,8 bis 0,9 M Enzymformen von der Säule eluiert wurden, welche im Normalblut fehlten.

## Beispiel 2

Die Bestimmungen gemäß Beispiel 1 wurden wiederholt, mit dem Unterschied, daß Blutplasma eines an Neuroblastom erkrankten Patienten verwendet wurde.

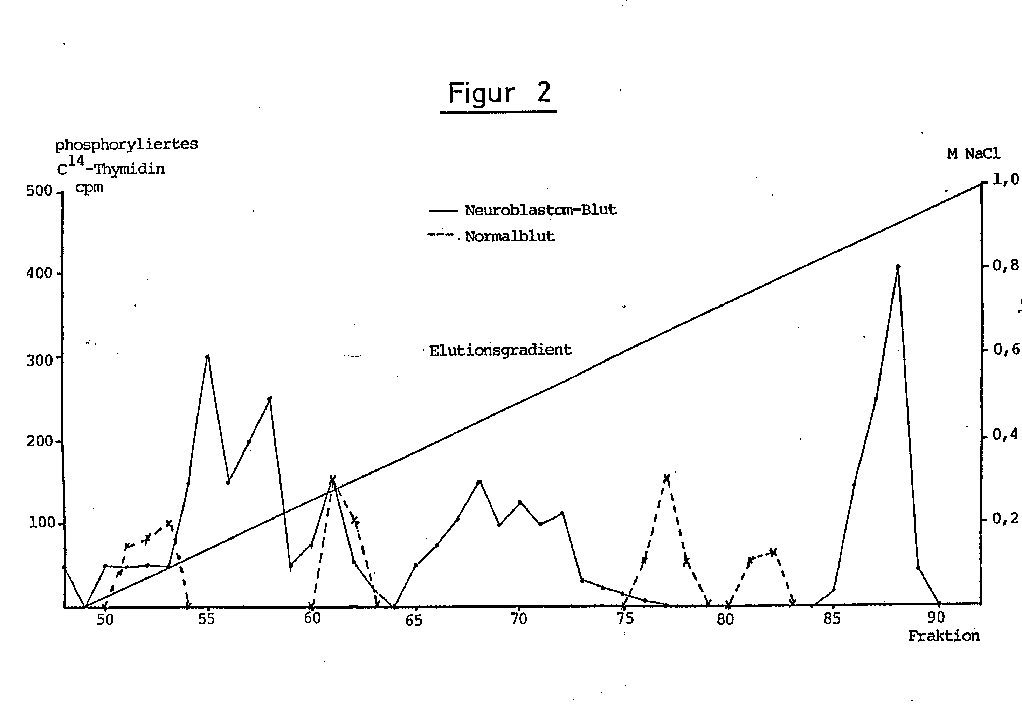

Die graphische Darstellung der Ergebnisse (vgl. Figur 2) zeigt, daß mit dem erfindungsgemäßen Verfahren auch in dem Blut eines an Neuroblastom erkrankten Patienten die für maligne Tumoren beim Menschen typischen Enzymformen nachgewiesen werden konnten.

## Beispiel 3

Männlichen ACI-Ratten (ca. 200 g Körpergewicht) wurden Morris Hepatome 9121 implantiert. Eine Woche nach der Transplantation wurde den Tieren Blut entnommen; zu diesem Zeitpunkt war ein Tumorwachstums mit herkömmlichen Verfahren nicht nachweisbar.

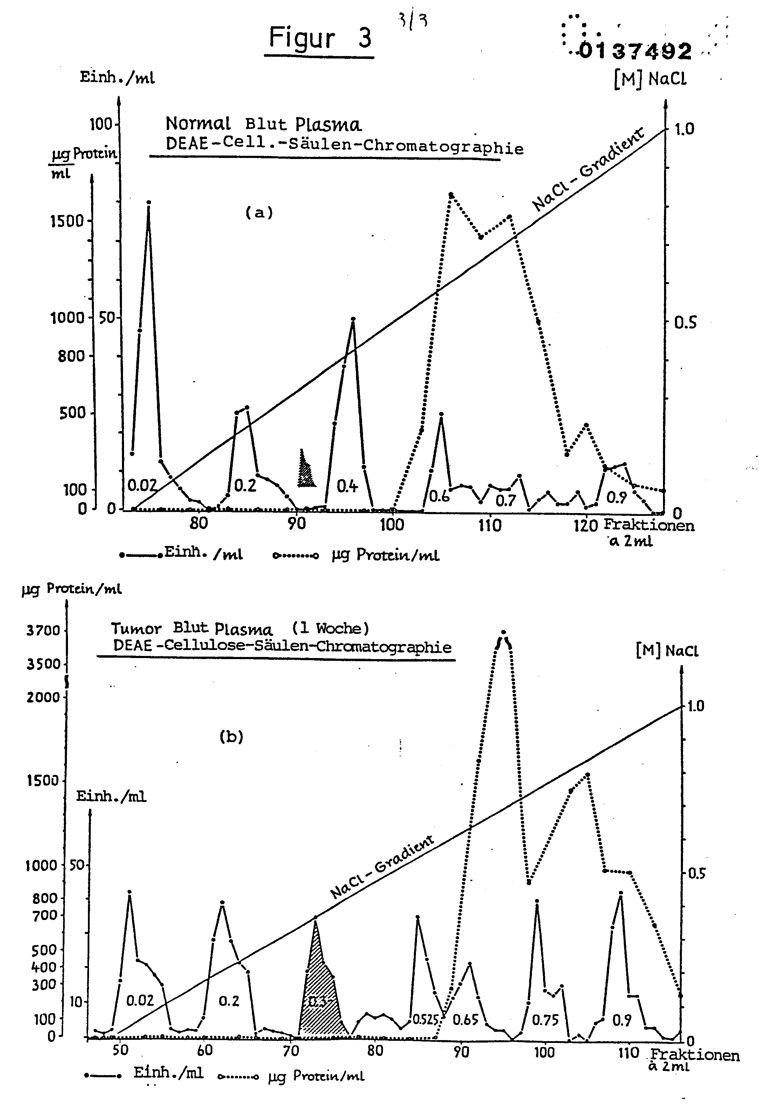

Das entnommene Blut wurde gemäß Beispiel 1 aufgearbeitet und die Ergebnisse der Bestimmungen der Nukleosid-Nukleotid-Phosphotransferasen wurden graphisch dargestellt (vgl. Figur 3 (b)). Der Vergleich mit dem Enzymmuster im Blut unbehandelter Tiere (vgl. Figur 3 (a)) zeigt, daß bereits eine Woche nach der Tumortransplantation im Blut der behandelten Tiere eine Enzymfraktion auftritt, die bei einer NaCl-Konzentration von O,3 M von der DEAE-Cellulose-Säule eluierbar ist und die im Blut unbehandelter Tiere nicht nachgewiesen werden kann.

**Patentansprüche für BE, CH, DE, FR, GB, IT, LI, NL, SE**

1.  Verfahren zur Frühdiagnose maligner Tumoren bei Säugetieren und Menschen, dadurch gekennzeichnet, daß man die im Blut vorhandenen Enzymformen der Nukleosid-Nukleotid-Phosphotransferasen bestimmt und das erhaltene Enzymmuster mit einem Standardmuster, erhalten aus dem Blut entsprechender gesunder Individuen, vergleicht.

2.  Verfahren nach Anspruch 1, dadurch gekennzeichnet, daß man zur Bestimmung des Enzymmusters auf an sich bekannte Weise das Blutplasma oder -serum gewinnt und dieses einer stufenweisen Aufarbeitung unterzieht, indem man das Blutplasma oder -serum zunächst mit 1N Essigsäure auf pH 4,5 einstellt, den Niederschlag bei 17.000 g abzentrifugiert, den Überstand mit konzentrierter KOH auf pH 7,5 einstellt und anschließend die Enzymform(en) zunächst durch Ausfällen und Zentrifugieren aus dem Blutplasma oder -serum isoliert, den erhaltenen Niederschlag löst, anschließend säulenchromatographisch fraktioniert und die erhaltenen Fraktionen hinsichtlich ihres Proteingehaltes sowie ihrer phosphorylierenden Aktivität bestimmt.

3.  Verfahren nach Anspruch 2, dadurch gekennzeichnet, daß man die Aufarbeitungsstufen jeweils in Morpholinopropansulfonsäure-Puffer (MOPS-Puffer) durchführt, dessen Molarität etwa 5 bis 15 mM und dessen pH-Wert etwa 6,9 bis 8 beträgt und der Dithiothreit (DTE) in einer Menge von 0,2 bis 1 mM enthält.

4. Verfahren nach Anspruch 2 oder 3, dadurch gekennzeichnet, daß man die Fällung in einer Mischung aus Blutplasma oder -serum und MOPS-Puffer im Verhältnis 1 : 2 durchführt, welche zu 33 % mit Ammoniumsulfat gesättigt ist und einen pH-Wert von 6,9 bis 8 aufweist.

5. Verfahren nach einem der Ansprüche 2 bis 4, dadurch gekennzeichnet, daß man die säulenchromatographische Fraktionierung auf Diethylaminoethylcellulose durch Elution mit einem linearen NaCl-Gradienten von 0,0 bis 1,0 M in MOPS-Puffer durchführt.

6. Verfahren nach einem der Ansprüche 1 bis 5, dadurch gekennzeichnet, daß man als Substrat für die Bestimmung der phosphorylierenden Aktivität der erhaltenen Fraktionen d-Thymidin verwendet.

7. Verfahren nach den Ansprüchen 1 bis 6, dadurch gekennzeichnet, daß man die in dem erhaltenen Enzymmuster im Vergleich zu dem Standardmuster zusätzlich vorhandene(n) Enzymform(en) für deren immunologischen Nachweis auf an sich bekannte Weise aus den die zusätzliche(n) Enzymform(en) enthaltenden Fraktionen isoliert.

8. Herstellung von monoklonalen oder polyklonalen Antikörpern für den immunologischen Nachweis der gemäß den Ansprüchen 1 bis 7 erhaltenen zusätzlichen Enzymform(en) im Blut von an malignen Tumoren erkrankten Säugetieren und Menschen, dadurch gekennzeichnet, daß man die gemäß Anspruch 7 isolierte(n) Enzymform(en) zur Bildung von Antikörpern befähigten Säugetieren verabreicht und auf an sich bekannte Weise gegen diese Enzymform(en) gerichtete Antikörper herstellt.

9.  Monoklonale Antikörper, dadurch erhältlich, daß man die nach Anspruch 7 isolierte zusätzlich vorhandene(n) Enzymform(en) zur Bildung von Antikörpern befähigten Säugetieren verabreicht und auf an sich bekannte Weise gegen die zusätzliche(n) Enzymform(en) gerichtete monoklonale Antikörper herstellt.

10. Polyklonale Antikörper, dadurch erhältlich, daß man die nach Anspruch 7 isolierte(n) zusätzlich vorhandene(n) Enzymform(en) zur Bildung von Antikörpern befähigten Säugetieren verabreicht und auf an sich bekannte Weise gegen die zusätzliche(n) Enzymform(en) gerichtete polyklonale Antikörper herstellt.

11. Verwendung der Antikörper nach den Ansprüchen 9 oder 10 zum immunologischen Nachweis der im Vergleich zu einem Standardmuster im Blut gesunder Säugetiere und Menschen im Blut von an malignen Tumoren erkrankten Individuen zusätzlich vorhandenen Enzymform(en) der Nukleosid-Nukleotid-Phosphotransferasen.

12. Testkit zur Durchführung des immunologischen Nachweises der im Vergleich zu einem Standardmuster aus dem Blut gesunder Säugetiere und Menschen im Blut von an malignen Tumoren erkrankten Individuen zusätzlich vorhandenen Enzymform(en) der Nukleosid-Nukleotid-Phosphotransferasen mit üblichen Hilfsmitteln für die Durchführung immunologischer Nachweise, gekennzeichnet durch mono- oder polyklonale Antikörper gemäß Anspruch 9 oder 10, welche zum Nachweis der Immunreaktion auf an sich bekannte Weise markiert sind.

13. Testkit zur Durchführung des immunologischen Nachweises der im Vergleich zu einem Standardmuster aus dem Blut gesunder Säugetiere und Menschen im Blut von an malignen Tumoren erkrankten Individuen zusätzlich vorhandenen Enzymform(en) der Nukleosid-Nukleotid-Phosphotransferasen mit üblichen Hilfsmitteln für die Durchführung immunologischer Nachweise, gekennzeichnet durch

a) Mittel zum Markieren der zusätzlichen Enzymform(en) im Blut von an malignen Tumoren erkrankten Säugetieren und Menschen in vivo oder in vitro für den Nachweis der Immunreaktion und

b) mono- oder polyklonale Antikörper gemäß Anspruch 9 oder 10.

0137492

Patentansprüche für AT -

1. Verfahren zur Frühdiagnose maligner Tumoren bei Säugetieren und Menschen, dadurch gekennzeichnet, daß
man die im Blut vorhandenen Enzymformen der Nukleosid-Nukleotid-Phosphotransferasen bestimmt und
das erhaltene Enzymmuster mit einem Standardmuster,
erhalten aus dem Blut entsprechender gesunder Individuen, vergleicht.

2. Verfahren nach Anspruch 1, dadurch gekennzeichnet,
daß man zur Bestimmung des Enzymmusters auf an sich
bekannte Weise das Blutplasma oder -serum gewinnt
und dieses einer stufenweisen Aufarbeitung unterzieht, indem man das Blutplasma oder -serum zunächst
mit 1N Essigsäure auf pH 4,5 einstellt, den Niederschlag bei 17.000 g abzentrifugiert, den Überstand
mit konzentrierter KOH auf pH 7,5 einstellt und anschließend die Enzymform(en) zunächst durch Ausfällen und Zentrifugieren aus dem Blutplasma oder
-serum isoliert, den erhaltenen Niederschlag löst,
anschließend säulenchromatographisch fraktioniert
und die erhaltenen Fraktionen hinsichtlich ihres
Proteingehaltes sowie ihrer phosphorylierenden Aktivität bestimmt.

3. Verfahren nach Anspruch 2, dadurch gekennzeichnet,
daß man die Aufarbeitungsstufen jeweils in Morpholinopropansulfonsäure-Puffer (MOPS-Puffer) durchführt, dessen Molarität etwa 5 bis 15 mM und dessen
pH-Wert etwa 6,9 bis 8 beträgt und der Dithiothreit
(DTE) in einer Menge von 0,2 bis 1 mM enthält.

0137492

*Erneuich*

4. Verfahren nach Anspruch 2 oder 3, dadurch gekennzeichnet, daß man die Fällung in einer Mischung aus Blutplasma oder -serum und MOPS-Puffer im Verhältnis 1 : 2 durchführt, welche zu 33 % mit Ammoniumsulfat gesättigt i und einen pH-Wert von 6,9 bis 8 aufweist.

5. Verfahren nach einem der Ansprüche 2 bis 4, dadurch gekennzeichnet, daß man die säulenchromatographische Fraktionierung auf Diethylaminoethylcellulose durch Elution mit einem linearen NaCl-Gradienten von 0,0 bis 1,0 M in MOPS-Puffer durchführt.

6. Verfahren nach einem der Ansprüche 1 bis 5, dadurch gekennzeichnet, daß man als Substrat für die Bestimmung der phosphorylierenden Aktivität der erhaltenen Fraktionen d-Thymidin verwendet.

7. Verfahren nach den Ansprüchen 1 bis 6, dadurch gekennzeichnet, daß man die in dem erhaltenen Enzymmuster im Vergleich zu dem Standardmuster zusätzlich vorhandene(n) Enzymform(en) für deren immunologischen Nachweis auf an sich bekannte Weise aus den die zusätzliche(n) Enzymform(en) enthaltenden Fraktionen isoliert.

8. Verfahren zur Herstellung von monoklonalen oder polyklonalen Antikörpern für den immunologischen Nachweis der gemäß den Ansprüchen 1 bis 7 erhaltenen zusätzlichen Enzymform(en) im Blut von an malignen Tumoren erkrankten Säugetieren und Menschen, dadurch gekennzeichnet, daß man die gemäß Anspruch 7 isolierte(n) Enzymform(en) zur Bildung von Antikörpern befähigten Säugetieren verabreicht und auf an sich bekannte Weise gegen diese Enzymform(en) gerichtete Antikörper herstellt.

*Österreich*

9. **Ver**wendung der nach Anspruch 8 hergestellten Antikörper zum immunologischen Nachweis der im Vergleich zu einem Standardmuster im Blut gesunder Säugetiere und Menschen im Blut von an malignen Tumoren erkrankten Individuen zusätzlich vorhandenen Enzymform(en) der Nukleosid-Nukleotid-Phosphotransferasen.

10. Testkit zur Durchführung des immunologischen Nachweises der im Vergleich zu einem Standardmuster aus dem Blut gesunder Säugetiere und Menschen im Blut von an malignen Tumoren erkrankten Individuen zusätzlich vorhandenen Enzymform(en) der Nukleosid-Nukleotid-Phosphotransferasen mit üblichen Hilfsmitteln für die Durchführung immunologischer Nachweise, gekennzeichnet durch mono- oder polyklonale Antikörper hergestellt gemäß Anspruch 8, welche zum Nachweis der Immunreaktion auf an sich bekannte Weise markiert sind.

11. Testkit zur Durchführung des immunologischen Nachweises der im Vergleich zu einem Standardmuster aus dem Blut gesunder Säugetiere und Menschen im Blut von an malignen Tumoren erkrankten Individuen zusätzlich vorhandenen Enzymform(en) der Nukleosid-Nukleotid-Phosphotransferasen mit üblichen Hilfsmitteln für die Durchführung immunologischer Nachweise, gekennzeichnet durch

   a) Mittel zum Markieren der zusätzlichen Enzymform(en) im Blut von an malignen Tumoren erkrankten Säugetieren und Menschen in vivo oder in vitro für den Nachweis der Immunreaktion und

   b) mono- oder polyklonale Antikörper hergestellt gemäß Anspruch 8.

Figur 1

Figur 2

Figur 3    3/3    0137492

Figure 3 (a): Normal Blut Plasma, DEAE-Cell.-Säulen-Chromatographie
Figure 3 (b): Tumor Blut Plasma (1 Woche), DEAE-Cellulose-Säulen-Chromatographie